# EUROPEAN PATENT SPECIFICATION

(11) **EP 2 641 594 B1**
(45) Date of publication and mention of the grant of the patent: **01.02.2017**
(21) Application number: 13160120.5
(22) Date of filing: 20.03.2013
(51) Int. Cl.: A61K 9/28, A61K 31/4439

(54) **Enteric coated solid pharmaceutical compositions for proton pump inhibitors**
Enterisch beschichtete feste pharmazeutische Zusammensetzungen für Protonenpumpeninhibitoren
Compositions pharmaceutiques solides gastro-résistantes enrobées pour inhibiteurs de pompe à protons

(30) Priority: 21.03.2012 TR 201203193
(43) Date of publication of application: 25.09.2013
(73) Proprietor: Sanovel Ilac Sanayi ve Ticaret A.S., 34460 Istanbul (TR)
(72) Inventor: Cifter, Ümit, 34460 Istanbul (TR); Türkyilmaz, Ali, 34460 Istanbul (TR); Mutlu, Onur, 34460 Istanbul (TR)
(74) Representative: Sevinç, Erkan

(56) References cited:
- EP-A1- 1 459 737
- EP-A2- 1 867 326
- EP-A2- 2 384 747
- US-A- 4 853 230
- SHIMIZU T ET AL: "FORMULATION STUDY FOR LANSOPRAZOLE FAST-DISINTEGRATING TABLET. II. EFFECT OF TRIETHYL CITRATE ON THE QUALITY OF THE PRODUCTS", CHEMICAL AND PHARMACEUTICAL BULLETIN, PHARMACEUTICAL SOCIETY OF JAPAN, JP, vol. 51, no. 9, 1 October 2003 (2003-10-01), pages 1029-1035, XP001174705, ISSN: 0009-2363, DOI: 10.1248/CPB.51.1029

## Description

### Field of Invention

The present invention is related to an enteric coated solid pharmaceutical composition for acid labile proton pump inhibitors.

### Background of the Invention

Proton pump inhibitors (PPI) are acid labile compounds useful for inhibiting gastric acid secretion. The problems arisen from this acid labile characteristic of these compounds is overcome by providing a formulation that dissolves in the intestines rather than the acidic enviroment of the stomach.

Using an antacid or an alkaline excipient with a PPI in the formulation is one method used in the art to reduce the acidity of the gastrointestinal environment. Another one is to provide an enteric coating for the composition comprising the acid labile PPI.

The main concern for these formulations is the design of the enteric coating. To achieve the desired dissolution profile the design of the composition and constituents of the enteric coating is very crucial. Two issues are the thickness of the coating, which must be adjusted to resist physical conditions, and the compounds used in the coating, which must be pharmaceutically compatible with the active ingredient and the excipients present in the coated composition to achieve a better stability.

It is known in the state of art that the enteric polymers with acidic nature are not compatible with the proton pump inhibitors and the alkaline excipients used in the formulation. To overcome this problem an inert intermediate layer may be introduced, such as in the prior art. In US 4,853,230 and WO 96/24338 enteric coated formulations with an alkaline active ingredient containing core and a separating layer are described.

EP 1 459 737 A1 discloses granules comprising a principal ingredient layer containing an acid-unstable medicament; an intermediate coating layer which is formed on the principal ingredient layer; and an enteric coating layer which is formed on the intermediate coating layer. In Example 2, for instance, disclosed is granules comprising lansoprazole and MgCO₃ spread onto the granules, an intermediate layer comprising low substituted HPMC and an enteric suspension.

Shimizu T. et al: "Formulation Study for Lansoprazole Fast Disintegrating Tablet II. Effect of Triethyl Citrate on the Quality of the Products', Chemical and Pharmaceutical Bulletin, Pharmaceutical Society of Japan, vol. 51, no. 9, 1 October 2003 (2003-10-01), pages 1029-1035*,* discloses granules comprising a core of inactive spheres surrounded by a layer of lansoprazole, an intermediate layer comprising HPMC and an enteric coating comprising a polymer composition and a plasticizer.

EP 2 384 747 A2 discloses granules comprising dexlansoprazole, a protective film and a single enteric coating.

Another role of the enteric coating is to provide a modified release. The delivery of the active ingredient to specific sites in the gastrointestinal tract is achieved by enteric coatings. These polymers can also prolong or extend the amount of active ingredient released from the dosage form.

However, modified release formulations of such can be prone to "dose dumping", which is one of the most important problems encountered with these formulations. The rapid release of the active ingredient at an early point causes the active ingredient concentration in the plasma to increase suddenly, which may lead to toxicity. Any cracks or thinned parts of the enteric coating layer might result in premature release of the active ingredient.

To overcome the issue of "dose dumping", the present invention provides a pharmaceutical formulation with an enteric coating that manifests a uniform disintegration characteristic. The pharmaceutical formulation of the invention also provides desired dissolution and improved stability characteristics.

### Description of the Invention

The object of the present invention is to provide an enteric coated composition for the acid labile proton pump inhibitors that achieves uniform disintegration in the gastrointestinal environment. To overcome previously mentioned problems and to achieve this object, the present invention provides a solid pharmaceutical composition for oral administration comprising a core comprising a proton pump inhibitor, an antacid and one or more pharmaceutically acceptable excipients; an intermediate layer, and an enteric coasting layer, according to claim 1.

As a further object of the invention, the pharmaceutical formulation of the invention also provides desired dissolution and improved stability characteristics.

The enteric coating layer of the present invention is designed to achieve maximum stability and uniform disintegration. The enteric coating layer of the invention is not affected by the momentary fluctuations in the pH of the gastrointestinal environment. Uniform disintegration suggests a controlled dissolution rate of the enteric coating layer in the gastrointestinal environment which induces a gradual release of the active ingredient; rather than a rapid release that results in dose dumping. "Uniform disintegration" of the enteric coating suggests a uniform dissolution rate of the coating at every point on the surface, thus uniform disintegration is observed throughout the surface layer, where dissolution of the solid particles of the enteric coating takes place. With "uniform disintegration" the coating thickness remains same throughout the coating layer as the coating dissolves, as a result no thinning regions of the coating, which might result in dose dumping, is observed.

The above mentioned "uniform disintegration" is achieved by the polymer composition of the present invention. This polymer composition comprises two enteric polymers.

The two polymers of the same type having different solubility characteristics of the invention are hydroxylpropylmethyl cellulose acetate succinate (HPMACS) polymers having different grades. HPMCAS polymers are commercially available in several grades, according to the viscosity and the pH at which the polymer dissolves (low, medium and high). In this present invention said grades of the HPMACS are determined according to their pH solubility as follows; low grade HPMCAS has a pH solubility ≥ 5.5, medium grade has a pH solubility ≥ 6.0 and high grade has a pH solubility ≥ 6.8.

The polymer composition of the invention comprises a higher grade HPMCAS, having pH solubility ≥ 6.8 or ≥6.0 and a lower grade HPMCAS, having a pH solubility ≥5.5 or ≥6.0. It has been surprisingly found that the enteric polymer composition comprising two HPMCAS with different grades provides a more uniform disintegration and dissolution characteristic for the enteric coating. Said higher grades and lower grades can be as follows;
i. a high grade hydroxylpropylmethyl cellulose acetate succinate, having pH solubility ≥6.8 and a medium grade hydroxylpropylmethyl cellulose acetate succinate, having pH solubility ≥6.0
ii. a high grade hydroxylpropylmethyl cellulose acetate succinate, having pH solubility ≥6.8 and a low grade hydroxylpropylmethyl cellulose acetate succinate, having pH solubility ≥5.5
iii. a medium grade hydroxylpropylmethyl cellulose acetate succinate, having pH solubility ≥6.0 and a low grade hydroxylpropylmethyl cellulose acetate succinate, having pH solubility ≥5.5

Using two HPMCAS polymers instead of two polymers of different types results in a more stable formulation since the same polymers of the same type have a better compatibility with each other and a similar solubility behaviour.

The preferred weight ratio of the higher grade HPMCAS to the lower grade HPMCAS is in the range of between 0.25 - 1.

The more preferred weight ratio of the higher grade HPMCAS to the lower grade HPMCAS is 1:3.

As a further embodiment of the invention, low grade HPMACS has an acetyl content in an amount of between 5.0 - 9.0 % by weight, succinoyl content in an amount between 14.0 - 18.0% by weight, methoxy content in an amount of between 20.0 - 24.0 % by weight and hydroxypropoxy content in an amount of between 5.0 - 9.0 % by weight. Medium grade HPMACS has an acetyl content in an amount of between 7.0 - 11.0 % by weight, succinoyl content in an amount between 10.0 - 14.0% by weight, methoxy content in an amount of between 21.0 - 25.0 % by weight and hydroxypropoxy content in an amount of between 5.0 - 9.0 % by weight. High grade HPMACS has an acetyl content in an amount of between 10.0 - 14.0 % by weight, succinoyl content in an amount between 4.0 - 8.0% by weight, methoxy content in an amount of between 22.0 - 26.0 % by weight and hydroxypropoxy content in an amount of between 6.0 - 10.0 % by weight.

As a further embodiment of the invention, it has also been found that using same types of enteric polymers such as different grades of HPMCAS, the use of anti-tacking agents such as talc and the like and wetting agents such as sodium lauryl sulfate and the like can be omitted. By providing an enteric coating without an anti-tacking agent or a wetting agent, a more stable formulation is obtained.

It has also been found that, when two HPMCAS polymers with different grades are used in the polymer composition the use of plasticizer is not obligatory and a plasticizer free enteric composition can also be provided. A plasticizer free enteric coating shows also improved stability characteristics.

However, the enteric coating layer of the invention may also contain a plasticizer. Plasticizers are used in the enteric coating solutions together with HPMCAS polymers to facilitate film formation. Yet, as a further embodiment, the release profile of the formulation might be adapted with the use of plasticizers. According to the desired release profile, the release of the PPI might be prolonged with the addition of plasticizer into the enteric coating.

The plasticizer used in the enteric coating is selected from the group of acetyltributyl citrate, acetyltriethyl citrate, cetyl alcohol, triacetin tributyl citrate and triethyl citrate. Preferably the plasticizer is triethyl citrate.

When an anti-tacking and wetting agent free formulation is designed, the amount of the plasticizer in the enteric coating must be limited to prevent excessive tackiness. It has been found that the optimized plasticizer is present in the enteric coating in an amount of 0.1-15% by weight when an anti-tacking and wetting agent free composition for enteric coating is provided for the polymer composition comprising two different grades of HPMCAS. The preferred plasticizer content of the enteric coating layer is 7-10%.

The core comprising a proton pump inhibitor, an antacid and one or more pharmaceutically acceptable excipients may be in the form of a granule, bead, pellet or tablet. Preferably the core is in the form of a tablet.

The proton pump inhibitors of the present invention can be selected from lansoprazole, omeprazole, pantoprazole and rabeprazole or their enantiomers, derivatives, pharmaceutically acceptable salts or hydrated forms. The preferred PPI compound used in the present invention is lansoprazole or omeprazole or their enantiomers, derivatives, pharmaceutically acceptable salts or hydrated forms.

According to this embodiment, lansoprazole enantiomer used in the present invention is dexlansoprazole and omeprazole enantiomer is esomeprazole.

The core comprises an antacid to reduce the acidity of the gastrointestinal environment. The antacid can be selected from the group of alginic acid, aluminum hydroxide, aluminum oxide, calcium carbonate, calcium silicate, magnesium carbonate, magnesium oxide, magnesium trisilicate, potassium bicarbonate, sodium bicarbonate and sodium carbonate. The preferred antacid of the present invention is magnesium oxide.

The core comprises one or more pharmaceutically acceptable excipients selected from the group comprising binders, diluents, fillers, lubricants, glidants, disintegrants, coloring agents or flavoring agents.

Suitable binders may comprise but not limited to methyl cellulose, ethyl cellulose, hydroxypropyl methyl cellulose, hydroxypropyl cellulose, sodium carboxymethyl cellulose, polyvinyl pyrrolidone, polyvinyl alcohol, polymethacrylates, starch, gelatin, alginic acid, sucrose and the like and mixtures thereof.

Suitable diluents and fillers may comprise but not limited to microcrystalline cellulose, cellulose, lactose, starch, calcium phosphates, calcium sulphates, mannitol, glucose, sucrose, sorbitol and the like and mixtures thereof.

Suitable lubricants may comprise but not limited to stearic acid, magnesium, calcium or sodium stearate, sodium stearyl fumarate, talc, waxes, liquid paraffin, and the like and mixtures thereof.

Suitable glidants may comprise but not limited to talc, aluminium silicate, colloidal silica, starch and the like and mixtures thereof.

Suitable disintegrants may comprise but not limited to alginic acid and salts, sodium carboxymethyl cellulose, hydroxypropyl methyl cellulose, starch, sodium starch glycolate, crosslinked polyvinyl pyrrolidone and the like and mixtures thereof.

According to the invention, an intermediate layer is applied over the core which provides a protective layer between the enteric polymers of the enteric coating layer and the active ingredient and excipient(s) present in the core. The intermediate layer of the present invention comprises a cellulosic polymer. The cellulosic polymer can be selected from the group of ethyl cellulose, hydroxyethyl cellulose, hydroxypropyl cellulose, hydroxypropylmethyl cellulose and methyl cellulose. The preferred cellulosic polymer of the intermediate layer is hydroxypropylmethyl cellulose.

The core applied with an intermediate layer is then coated with the previously described enteric coating compositions of the invention comprising a polymer composition. The present invention is further defined by reference to the following examples. Although the examples are not intended to limit the scope of the present invention, they should be considered in the light of the description detailed above.

### Example 1

### Enteric coating solution for dexlansoprazole tablet

Granules containing dexlansoprazole and magnesium oxide are mixed with excipients and compacted into tablets. The tablets are then coated with a hydroxypropylmethyl cellulose containing coating solution. An enteric coating solution is then prepared by mixing a high grade HPMCAS and a medium grade HPMCAS in the ratio of 3:1. Triethyl citrate is added to the solution. The enteric coating solution is then applied on the previously coated tablets.

### Example 2

### Enteric coating solution for esomeprazole tablet

Granules containing esomeprazole and magnesium oxide are mixed with excipients and compacted into tablets. The tablets are then coated with a hydroxypropylmethyl cellulose containing coating solution. An enteric coating solution is then prepared by mixing a high grade HPMCAS and a medium grade HPMCAS in a ratio of 3:1. The enteric coating solution is then applied on the previously coated tablets.

## Claims

1. A solid pharmaceutical composition for oral administration comprising;
i. a core comprising a proton pump inhibitor, an antacid and one or more pharmaceutically acceptable excipients
ii. an intermediate layer, and
iii. an enteric coating layer
**characterized in that**,
a. the intermediate layer comprises a cellulosic polymer,
b. the enteric coating layer comprises a polymer composition and optionally a plasticizer, said polymer composition comprising two enteric polymers having different solubility characteristics which are;
- hydroxypropylmethyl cellulose acetate succinate having a higher grade, having pH solubility ≥ 6.8 or ≥6.0 and
- hydroxylpropylmethyl cellulose acetate succinate having a lower grade, having a pH solubility ≥5.5 or ≥6.0.

2. The solid pharmaceutical composition according to claim 1, wherein the proton pump inhibitor is selected from the group of lansoprazole, omeprazole, pantoprazole and rabeprazole or their enantiomers, pharmaceutically acceptable salts or hydrated forms.

3. The solid pharmaceutical composition according to claim 2, wherein the proton pump inhibitor is dexlansoprazole.

4. The solid pharmaceutical composition according to claim 2, wherein the proton pump inhibitor is esomeprazole.

5. The solid pharmaceutical composition according to claim 1, wherein the core is in the form of a granule, bead, pellet or tablet.

6. The solid pharmaceutical composition according to claim 1, wherein the antacid is selected from alginic acid, aluminum hydroxide, aluminum oxide, calcium carbonate, calcium silicate, magnesium carbonate, magnesium oxide, magnesium trisilicate, potassium bicarbonate, sodium bicarbonate and sodium carbonate.

7. The solid pharmaceutical composition according to claim 1, wherein the cellulosic polymer is selected from ethyl cellulose, hydroxyethyl cellulose, hydroxypropyl cellulose, hydroxypropylmethyl cellulose and methyl cellulose.

8. The solid pharmaceutical composition according to claim 1, wherein the two polymers are selected from one of the following:
i. a high grade hydroxylpropylmethyl cellulose acetate succinate, having pH solubility ≥6.8 and a medium grade hydroxylpropylmethyl cellulose acetate succinate, having pH solubility ≥6.0
ii. a high grade hydroxylpropylmethyl cellulose acetate succinate, having pH solubility ≥6.8 and a low grade hydroxylpropylmethyl cellulose acetate succinate, having pH solubility ≥5.5
iii. a medium grade hydroxylpropylmethyl cellulose acetate succinate, having pH solubility ≥6.0 and a low grade hydroxylpropylmethyl cellulose acetate succinate, having pH solubility ≥5.5

9. The solid pharmaceutical composition according to claim 1 and 8, wherein the weight ratio of the higher grade hydroxylpropylmethyl cellulose acetate succinate to the lower grade hydroxylpropylmethyl cellulose acetate succinate is in the range of 0.25-1.

10. The solid pharmaceutical composition according to claim 9, wherein the weight ratio of the higher grade HPMCAS to the lower grade HPMCAS is 1:3.

11. The solid pharmaceutical composition according to claim 1, wherein the plasticizer is selected from acetyltributyl citrate, acetyltriethyl citrate, cetyl alcohol, triacetin tributyl citrate and triethyl citrate.

12. The solid pharmaceutical composition according to claim 11, wherein the plasticizer is present in the enteric coating layer in an amount of 0.1-15% by weight.

13. The solid pharmaceutical composition according to claim 1, wherein the one or more pharmaceutically acceptable excipients are selected from the group comprising binders, diluents, fillers, lubricants, glidants, stabilizing agents, coloring agents, flavouring agents and mixtures thereof.

## Patentansprüche

1. Feste pharmazeutische Zusammensetzung für die orale Verabreichung, umfassend:
i. einen Kern, umfassend einen Protonenpumpenhemmer, ein Antazidum und einen oder mehrere pharmazeutisch verträgliche Hilfsstoffe,
ii. eine Zwischenschicht und
iii. eine magensaftresistente Überzugsschicht,
**dadurch gekennzeichnet, dass**
a. die Zwischenschicht ein Cellulosepolymer umfasst,
b. die magensaftresistente Überzugsschicht eine Polymerzusammensetzung und gegebenenfalls einen Weichmacher umfasst, wobei die Polymerzusammensetzung zwei magensaftresistente Polymere mit unterschiedlichen Löslichkeitseigenschaften umfasst, die
- höhergradiges Hydroxypropylmethylcelluloseacetatsuccinat mit einer pH-Löslichkeit von ≥ 6,8 oder ≥ 6,0 und
- niedergradigeres Hydroxypropylmethylcelluloseacetatsuccinat mit einer pH-Löslichkeit von ≥ 5,5 oder ≥ 6,0
sind.

2. Feste pharmazeutische Zusammensetzung gemäß Anspruch 1, wobei der Protonenpumpenhemmer ausgewählt ist aus der Gruppe von Lansoprazol, Omeprazol, Pantoprazol und Rabeprazol oder ihren Enantiomeren, pharmazeutisch verträglichen Salzen und hydratisierten Formen.

3. Feste pharmazeutische Zusammensetzung gemäß Anspruch 2, wobei der Protonenpumpenhemmer Dexlansoprazol ist.

4. Feste pharmazeutische Zusammensetzung gemäß Anspruch 2, wobei der Protonenpumpenhemmer Esomeprazol ist.

5. Feste pharmazeutische Zusammensetzung gemäß Anspruch 1, wobei der Kern in der Form eines Granulatkorns, eines Kügelchens, eines Pellets oder einer Tablette vorliegt.

6. Feste pharmazeutische Zusammensetzung gemäß Anspruch 1, wobei das Antazidum ausgewählt ist aus Alginsäure, Aluminiumhydroxid, Aluminiumoxid, Calciumcarbonat, Calciumsilicat, Magnesiumcarbonat, Magnesiumoxid, Magnesiumtrisilicat, Kaliumbicarbonat, Natriumbicarbonat und Natriumcarbonat.

7. Feste pharmazeutische Zusammensetzung gemäß Anspruch 1, wobei das Cellulosepolymer ausgewählt ist aus Ethylcellulose, Hydroxyethylcellulose, Hydroxypropylcellulose, Hydroxypropylmethylcellulose und Methylcellulose.

8. Feste pharmazeutische Zusammensetzung gemäß Anspruch 1, wobei die beiden Polymere ausgewählt sind aus einem der folgenden:
i. einem hochgradigen Hydroxypropylmethylcelluloseacetatsuccinat mit einer pH-Löslichkeit von ≥ 6,8 und einem mittelgradigen Hydroxypropylmethylcelluloseacetatsuccinat mit einer pH-Löslichkeit von ≥ 6,0,
ii. einem hochgradigen Hydroxypropylmethylcelluloseacetatsuccinat mit einer pH-Löslichkeit von ≥ 6,8 und einem niedergradigen Hydroxypropylmethylcelluloseacetatsuccinat mit einer pH-Löslichkeit von ≥ 5,5,
iii. einem mittelgradigen Hydroxypropylmethylcelluloseacetatsuccinat mit einer pH-Löslichkeit von ≥ 6,0 und einem niedergradigen Hydroxypropylmethylcelluloseacetatsuccinat mit einer pH-Löslichkeit von ≥ 5,5.

9. Feste pharmazeutische Zusammensetzung gemäß Anspruch 1 und 8, wobei das Gewichtsverhältnis des höhergradigen Hydroxypropylmethylcelluloseacetatsuccinats zu dem niedergradigeren Hydroxypropylmethylcelluloseacetatsuccinat in dem Bereich von 0,25-1 liegt.

10. Feste pharmazeutische Zusammensetzung gemäß Anspruch 9, wobei das Gewichtsverhältnis des höhergradigen HPMCAS zu dem niedergradigeren HPMCAS 1:3 beträgt.

11. Feste pharmazeutische Zusammensetzung gemäß Anspruch 1, wobei der Weichmacher ausgewählt ist aus Acetyltributylzinn, Acetyltriethylcitrat, Cetylalkohol, Triacetintributylcitrat und Triethylcitrat.

12. Feste pharmazeutische Zusammensetzung gemäß Anspruch 11, wobei der Weichmacher in der magensaftresistenten Überzugsschicht in einer Menge von 0,1-15 Gew.-% vorhanden ist.

13. Feste pharmazeutische Zusammensetzung gemäß Anspruch 1, wobei der eine oder die mehreren pharmazeutisch verträglichen Hilfsstoffe ausgewählt sind aus der Gruppe umfassend Bindemittel, Verdünnungsmittel, Füllstoffe, Gleitmittel, Fließregulierungsmittel, Stabilisierungsmittel, Farbstoffe, Aromastoffe und Gemische davon.

## Revendications

1. Une composition pharmaceutique solide pour administration orale comprenant ;
i. un noyau comprenant un inhibiteur de la pompe à protons, un antiacide et un ou plusieurs excipients pharmaceutiquement acceptables
ii. une couche intermédiaire, et
iii. une couche d'enrobage entérique
**caractérisée en ce que**,
a. la couche intermédiaire comprend un polymère cellulosique,
b. la couche d'enrobage entérique comprend une composition polymère et éventuellement un plastifiant, ladite composition polymère comprenant deux polymères entériques présentant des caractéristiques de solubilité différentes qui sont ;
- de l'acétate-succinate d'hydroxypropylméthylcellulose de grade supérieur, ayant un pH de solubilité ≥ 6,8 ou ≥ 6,0 et
- de l'acétate-succinate d'hydroxypropylméthylcellulose de grade inférieur, ayant un pH de solubilité ≥ 5,5 ou ≥ 6,0.

2. La composition pharmaceutique solide selon la revendication 1, où l'inhibiteur de la pompe à protons est sélectionné parmi le groupe constitué de lansoprazole, oméprazole, pantoprazole et rabéprazole ou leurs enantiomères, leurs sels ou leurs formes hydratées pharmaceutiquement acceptables.

3. La composition pharmaceutique solide selon la revendication 2, où l'inhibiteur de la pompe à protons est du dexlansoprazole.

4. La composition pharmaceutique solide selon la revendication 2, où l'inhibiteur de la pompe à protons est de l'ésoméprazole.

5. La composition pharmaceutique solide selon la revendication 1, où le noyau est sous forme de granulé, de bille, de pastille ou de comprimé.

6. La composition pharmaceutique solide selon la revendication 1, où l'antiacide est sélectionné parmi l'acide alginique, l'hydroxyde d'aluminium, l'oxyde d'aluminium, le carbonate de calcium, le silicate de calcium, le carbonate de magnésium, l'oxyde de magnésium, le trisilicate de magnésium, le bicarbonate de potassium, le bicarbonate de sodium et le carbonate de sodium.

7. La composition pharmaceutique solide selon la revendication 1, où le polymère cellulosique est sélectionné parmi l'éthylcellulose, l'hydroxyéthylcellulose, l'hydroxypropylcellulose, l'hydroxypropylméthylcellulose et la méthylcellulose.

8. La composition pharmaceutique solide selon la revendication 1, où les deux polymères sont sélectionnés parmi l'un des suivants :
i. un acétate-succinate d'hydroxypropylméthylcellulose de grade supérieur, ayant un pH de solubilité ≥ 6,8 et un acétate-succinate d'hydroxypropylméthylcellulose de grade intermédiaire, ayant un pH de solubilité ≥ 6,0
ii. un acétate-succinate d'hydroxypropylméthylcellulose de grade supérieur, ayant un pH de solubilité ≥ 6,8 et un acétate-succinate d'hydroxypropylméthylcellulose de grade inférieur, ayant un pH de solubilité ≥ 5,5
iii. un acétate-succinate d'hydroxypropylméthylcellulose de grade intermédiaire, ayant un pH de solubilité ≥ 6,0 et un acétate-succinate d'hydroxypropylméthylcellulose de grade inférieur, ayant un pH de solubilité ≥ 5,5

9. La composition pharmaceutique solide selon les revendications 1 et 8, où le rapport pondéral de l'acétate-succinate d'hydroxypropylméthylcellulose de grade supérieur à l'acétate-succinate d'hydroxypropylméthylcellulose de grade inférieur est dans l'intervalle 0,25 - 1.

10. La composition pharmaceutique solide selon la revendication 9, où le rapport pondéral de l'HPMCAS de grade supérieur à l'HPMCAS de grade inférieur est 1:3.

11. La composition pharmaceutique solide selon la revendication 1, où le plastifiant est sélectionné parmi le citrate d'acétyltributyle, le citrate d'acétyltriéthyle, l'alcool cétylique, la triacétine, le citrate de tributyle et le citrate de triéthyle.

12. La composition pharmaceutique solide selon la revendication 11, où le plastifiant est présent dans la couche d'enrobage entérique à raison de 0,1 à 15 % en poids.

13. La composition pharmaceutique solide selon la revendication 1, où le ou les excipient(s) pharmaceutiquement acceptable(s) sont sélectionnés parmi le groupe comprenant des liants, des diluants, des charges, des lubrifiants, des agents de glissement, des agents stabilisateurs, des colorants, des aromatisants et des mélanges de ceux-ci.
